# EUROPEAN PATENT SPECIFICATION

(11) **EP 1 863 379 B1**
(45) Date of publication and mention of the grant of the patent: **23.03.2011**
(21) Application number: 06717415.1
(22) Date of filing: 03.01.2006
(51) Int. Cl.: A61B 5/00, A61B 6/00, G06T 7/40, G06Q 30/00, G01J 3/46

(54) **COSMETIC COLOR DETERMINATE SYSTEM WITH INFRARED SENSING**
KOSMETISCHES FARBBESTIMMUNGSSYSTEM MIT INFRAROTMESSUNG
SYSTEME DE DETERMINATION DE COULEURS COSMETIQUES AVEC DETECTION INFRAROUGE

(30) Priority: 05.01.2005 US 641696 P
(43) Date of publication of application: 12.12.2007
(73) Proprietor: Aevora Beauty Concepts, LLC., La Jolla CA 92037 (US)
(72) Inventor: LADJEVARDI, Mahmoud, La Jolla, CA 92037 (US); FORSTER, Albert, Irvine, CA 92612 (US)
(74) Representative: Dr. Graf & Partner AG
(86) International application number: PCT/US2006/000207
(87) International publication number: WO 2006/074241

(56) References cited:
- WO-A-01/23850
- WO-A-03/012728
- US-A- 6 147 761
- US-B1- 6 178 341
- US-B1- 6 437 866
- US-B1- 6 437 866

## Description

### Field of the Invention

The field of the invention is hair color determination.

### Background

Researchers have been trying to perfect computer aided determination of hair and skin color for many years. There is a forensic application, where individual fibers of hair and pieces of skin are subjected to detailed spectral analysis. The goal is to make either an exact match with a target individual, or to rule out the match. There are also medical applications, where the spectral characteristics of skin or mucous membrane is used to detect early stages of cancer. In all of those applications it is desirable to have a full spectral analysis. In some sense, that makes the determination easier, because one can use existing spectral equipment and techniques.

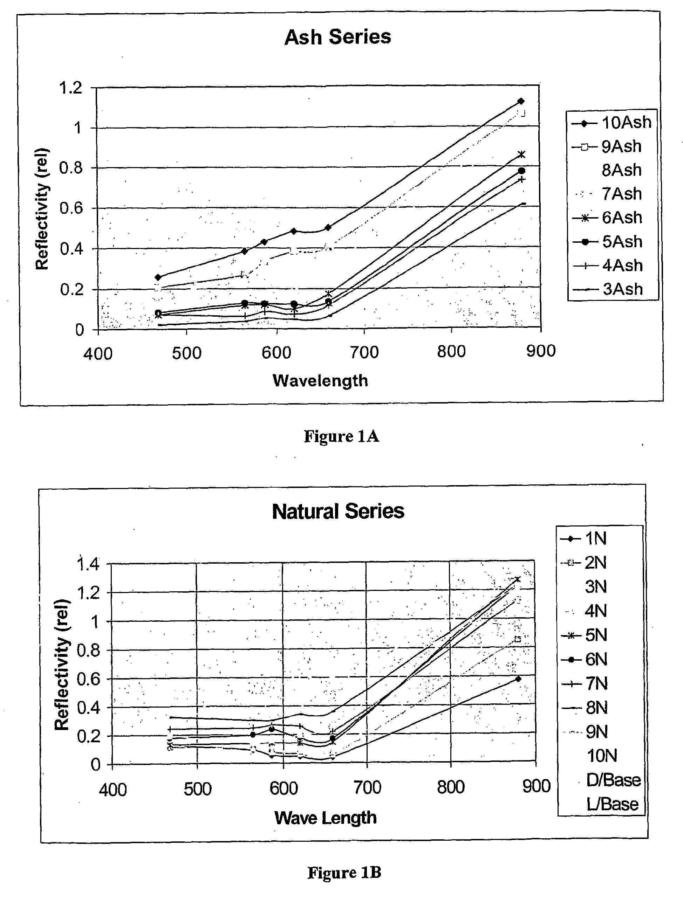

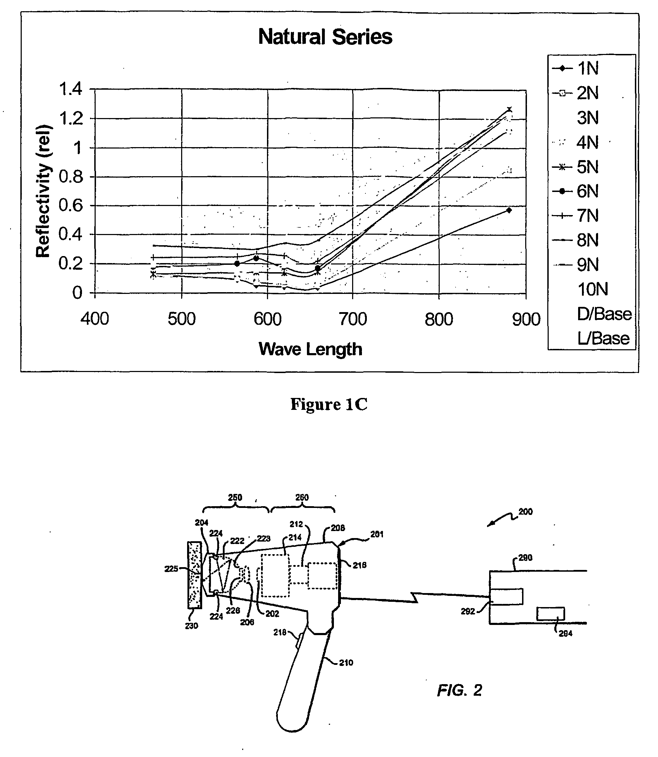

For cosmetic applications, the task is more difficult. In cosmetics, one is more interested in the *appearance* of the hair or skin to a human observer than in determining the exact spectral characteristics. Moreover, the main contemplated use is not to establish or reject an exact match, but to select a corresponding color from a database, which can then be utilized to select a make-up foundation, hair coloring, or other cosmetic product. Existing techniques for determining cosmetic appearance have not be particularly successful. In technical terms, some of these shortcomings can be summarized as follows:
a) Many existing attempts require transformation from one color space to another. For example, if a camera uses the CYMG color separation technique, it will often require color space transformation to end up with a RGB color image. Since not all pixels in the sensor provide all the color information, a scan conversion algorithm is required to obtain a complete CYMG bitmap. At sharp luminance changes in the image data (edges of hair) significant interpolation errors can occur. In addition, the color space transformation itself generally translates into reduced discrimination power for color matching measurements.
b) In most existing systems, only three relative wide color bands are used to determine the hair color. Fig. 1 shows a typical response for a CYMG sensor. The average value in these color bands are used as signature for color matching. However, differences within these bands for hair samples are averaged out and become smaller compared to the background noise. When these differences are the signatures used for discriminating between different hair samples, the color matching can be readily corrupted. As a simple example, "salt and pepper" hair might well appear to the machine as a light brown. Again the CYMG color space and its conversion into RGB worsens the situation because differences of measurements are required which increases the measurement errors.
c) The gray scale resolution after all the camera image processing is significantly less than bits of gray-scale in the blue, green and red color bitmaps. Gray scale value are the direct parameter used in the color matching algorithm.
d) Color expansion algorithms typically use a scan conversion technique which cross-mixes pixels and reduces the pixel resolution. Since pixel resolution is different in the red, green and blue channels, fine structured features in the image can be significantly misrepresented after all be correction data manipulation.
e) Color bands of the camera and the illumination source are not matched to the reflectivity changes in hair samples.

Document WO 03/012728 A1 discloses a cosmetic color analysis system according to the preamble of claim 1 that analyzes the color of hair to determine cosmetic hair color. A major drawback of such a system is that the accuracy to determine hair color is still limited.

U.S. Patent No. 6,807,297 teaches a cosmetic color determinate system that determines hair color with greater accuracy than previous systems. This and all other referenced patents and applications are incorporated herein by reference in their entirety. Where a definition or use of a term in an incorporated reference is inconsistent or contrary to the definition of that term provided herein, the definition of that term provided herein applies and the definition of that term in the reference does not apply.

Among other things, the '297 patent teaches splitting the visual field into many smaller subsets, averaging the color values for the pixels within each of the subsets, and then averaging the color values of the subsets. Despite the high degree of accuracy achieved, even systems according the '297 teachings have difficulty distinguishing various close colors, especially different strawberry or other reddish blond colors. The chief reason is that the '297 patent, as well as all other known cosmetic color determination devices and methods focus entirely on the visible wavelengths.

It turns out that the focus on visible wavelengths is inherently problematic because the greatest differences in *close* hair and skin colors are found outside the visible regions, in the infrared. These results are quite unexpected because infrared (IR) is by definition outside the normal range of visible light for humans. One of ordinary skill would therefore assume that IR wavelengths would be useless in determining cosmetic colors, i.e. the colors that a human observer can see. But the opposite is true. It turns out that inclusion of IR in the determination of hair color provides a significant benefit in distinguishing close colors.

In Figure 1A the differences among the different ash hair colors are more pronounced above 800 nm than in the 450 - 800 nm region. This is also apparent in the so-called "natural series" shown in Figure 1B. And the phenomena is even more pronounced in the very samples that prior devices had the most trouble distinguishing (see Figure 1C).

Thus, there is still a need to even further increase the accuracy of electronically assisted cosmetic (hair and skin) color determination, and there is an especial need for devices and methods that utilize wavelengths outside the visual wavelengths.

### Summary of The Invention

The present invention provides systems that use infrared ("IR") wavelengths to assist in cosmetic determination of hair color as defined in claim 1. The embodiments include a light collector that has significant sensitivity to light waves having a wavelength above 700 nm, and in various contemplated embodiments the light collector may have significant sensitivity to light waves above 750, 800, 850 nm, 900 nm, 1000 nm and/or 1100 nm.

Various objects, features, aspects and advantages of the present invention will become more apparent from the following detailed description of preferred embodiments of the invention, along with the accompanying drawings in which like numerals represent like components.

### Brief Description of The Drawing

Fig. 1A is a graph showing frequency absorption of different ash hair colors.
Fig. 1B is a graph showing frequency absorption of different "natural series" hair colors.
Fig. 1C is a graph showing frequency absorption of specific hair samples.
Figure 2 is a schematic of a cosmetic color determination system that includes a pixel capture device having a collector.
Figure 3 is a schematic showing how data from a field of pixels 110 is.summarized into R (red), G (green), B (blue), and IR (infrared) values 122 for each of a plurality of proper subsets 120 of the field 110.

### Detailed Description

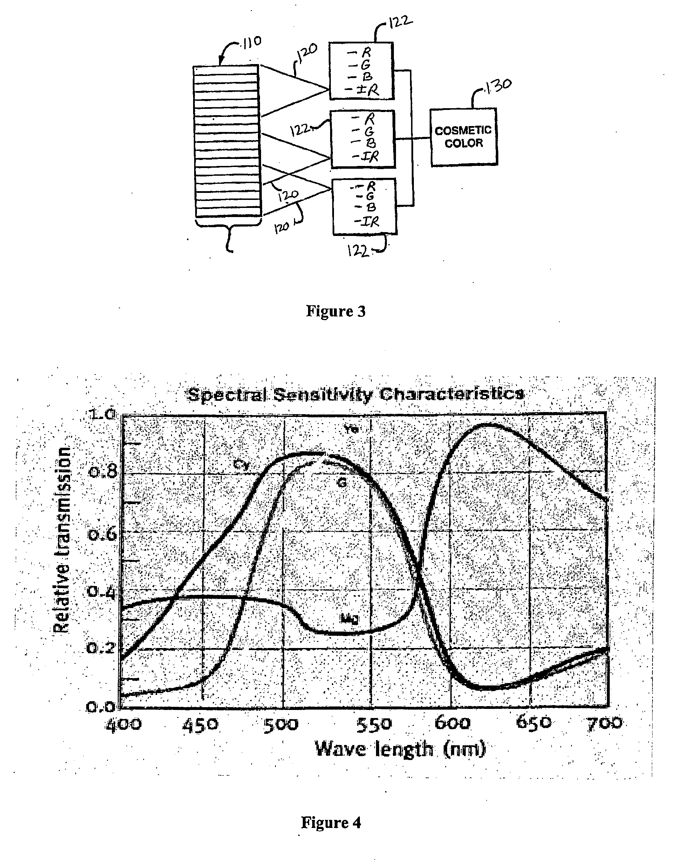

**Figure 3** is a schematic showing how data from a field of pixels 110 is summarized into R (red), G (green), B (blue), and IR (infrared) values 122 for each of a plurality ofproper subsets 120 of the field 110. The various R, G, B, and IR values 122 are interpreted into a cosmetic color 130.

In **Figure 2**, a cosmetic color determination system 200 includes a pixel capture device 201 having a collector 202. It is important to recognize that although IR values are not within the generally accepted range of visible light, their impact on a cosmetic color determination is significant and therefore collector 202 has significant sensitivity above 700 nm. As used herein, the term "significant sensitivity" means that the collector collects light waves in a designated range more than to a nominal extent. In preferred embodiments, significant sensitivity is achieved by doping portions of a CCD (Charge Coupled Device) or other pixel capture device to enhance IR sensitivity. In especially preferred embodiments, the pixel capture device has sensitivity near 750, 800, 850, 900, 950, 1000, 1100, and/or 1200 that is comparable to sensitivity of the device in one or more portions of the visible spectrum.

In practice, the image collector 202 receives waves that are in the visible light range and waves that are above the visible range. Typically, the light waves are digitally represented and then communicated to an analytical component such as the data processing unit 290 or a distal computer (not shown). The analytical component utilizes the data, including infrared data, to determine hair color, skin color, or another cosmetic color.

Analysis of the wavelength data can be undertaken in any suitable manner. This includes averaging an entire field, and alternatively examining subsets of a field. It is especially contemplated that software and/or hardware can be used to overweight particular wavelengths or ranges of wavelengths in the analysis. From a hardware standpoint, the analysis can be performed in or adjacent to a camera, in a computer, PDA or other computing device, and communication of the raw data to the computing device can be hardwired, wireless, and so forth. Raw data can be presented in an RGB type format, or in any other suitable color space.

Analytical results can be presented to a user or customer through a CRT, flat screen, PDA, or any other suitable monitor, can be delivered audibly, and can be printed on any manner of printer. Of particular interest are embodiments in which an image is made of the hair color, (possibly including also the subject's face) and alternative colorations could be presented with reference to particular hair coloring products.

It is still further contemplated although not forming part of the invention, that detection of IR spectra can be used to improve determination of skin color. In practice, the operation would be similar; namely a CCD or other device with sensitivity in some portion of the IR region, especially near IR, and use of that information in analyzing the skin color. The use of IR in determination of skin color is expected to be especially useful in analyzing lighter color skin surfaces, including mucous membranes. It is especially contemplated that a skin color determinate system would include a hand-held adaptor that carries the light collector, and is sized and dimensioned for use on facial skin, or alternatively on a mucous membrane. Such an adaptor, may for example, advantageously have a elongated pen shape for application to the cheek of a person, or for insertion into a body orifice.

It is also contemplated that IR data can be produced using other techniques besides those discussed above. For example, instead of having pixels with particularly high sensitivity to IR wavelengths, it is contemplated that a more generic sensor may be used in conjunction with one or more light sources that provide particular wavelengths or bands of wavelengths that include IR.

Still another contemplated embodiment takes advantage of the fact that many cyan or blue pigments have relatively high transmissivity in both blue and IR regions. This phenomena, demonstrated in the chart below, allows one to use a normal CYMG or RGB camera. But instead of ignoring the IR data, one expressly utilizes the IR data for purposes of cosmetic hair and skin color determinations. Indeed, in view of the charts above, one would most likely ignore the blue data provided by those pixels. See Figure 4.

Certainly it should be appreciated that cosmetic color determination systems and methods as discussed herein could be utilized with appropriate software and databases to select a hair coloring or skin care product from one of the databases. Preferred hair coloring products that could be selected include dyes, gray cover-ups, and conditioners. Preferred skin care products that could be selected include foundations and highlights. In all of these cases it is highly probably that a manufacturer would want to use a database that directs a customer to a proprietary product.

In addition, it should be apparent to those skilled in the art that many more modifications besides those already described are possible. Moreover, in interpreting the disclosure, all terms should be interpreted in the broadest possible manner consistent with the context. In particular, the terms "comprises" and "comprising" should be interpreted as referring to elements, components, or steps in a non-exclusive manner, indicating that the referenced elements, components, or steps could be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

## Claims

1. A cosmetic color determination system (200) comprising: a light collector (202) having significant sensitivity in visible red and green wavelengths and further significant sensitivity above 700 nm; and an analytical component (290) that utilizes the visible wavelengths data from the light collector (202) to determine cosmetic hair color, **characterised in that** the analytical component (290) also utilizes infrared data from the light collector (202) to determine cosmetic hair color.

2. The system of claim 1, wherein the light collector (202) has the significant sensitivity in visible red and green wavelengths and to light waves above 800 nm.

3. The system of claim 1 or 2, wherein the light collector (202) also has a significant sensitivity in visible blue wavelengths, and the analytical component (290) utilizes the visible wavelengths and the infrared data in determining the hair color.

4. The system of one of the preceding claims, further comprising a mechanism for overweighting particular wavelengths or ranges of wavelengths in determining the hair color.

5. A hair product recommending system that includes the cosmetic color determination system of one of claims 1 to 4, a database of hair coloring products, and software that utilizes information from the cosmetic color determination system to select a hair coloring product from the database.

6. A skin product recommending system that includes the cosmetic color determination system of one of claims 1 or 2, a database of skin care products, and software that utilizes information from the cosmetic color determination system to select a skin care product from the database.

7. The skin product recommending system of claim 6, wherein the selected skin care product comprises a cosmetics foundation.

## Patentansprüche

1. Ein kosmetisches Farbbestimmungssystem (200) umfassend: einen Lichtkollektor (202) aufweisend eine signifikante Sensitivität bei sichtbaren roten und grünen Wellenlängen und weiter eine signifikante Sensitivität oberhalb 700 nm; und eine analytische Komponente (290) welche die sichtbaren Wellelängendaten des Lichtkollektors (202) verwendet um die kosmetische Haarfarbe zu bestimmen, **dadurch gekennzeichnet, dass** die analytische Komponente (290) auch Infrarotdaten des Lichtkollektors (202) verwendet zur Bestimmung der kosmetischen Haarfarbe.

2. Das System gemäss Anspruch 1, wobei der Lichtkollektor (202) die signifikante Sensitivität bei sichtbaren roten und grünen Wellenlängen und bei Lichtwellen oberhalb 800 nm aufweist.

3. Das System gemäss Anspruch 1 oder 2, wobei der Lichtkollektor (202) zudem eine signifikante Sensitivität bei sichtbaren blauen Wellenlängen aufweist, und wobei die analytische Komponenten (290) die sichtbaren Wellenlängen und die Infrarotdaten verwendet bei der Bestimmung der Haarfarbe.

4. Das System gemäss einem der vorhergehenden Ansprüche, weiter umfassend eine Vorrichtung zum Übergewichten bestimmter Wellenlängen oder Bereiche von Wellenlängen bei der Bestimmung der Haarfarbe.

5. Ein Haarproduktempfehlungssystem welches das kosmetische Farbbestimmungssystem gemäss einem der Ansprüche 1 bis 4 umfasst, eine Datenbank von Haarfärbungsprodukten, und Software welche Informationen vom kosmetischen Farbbestimmungssystem nutzt um ein Haarfärbungsprodukt aus der Datenbank zu wählen.

6. Ein Hautproduktempfehlungssystem welches das kosmetische Farbbestimmungssystem gemäss einem der Ansprüche 1 oder 2 umfasst, eine Datenbank von Hautpflegeprodukten, und Software welche Informationen vom kosmetischen Farbbestimmungssystem nutzt um ein Hautpflegeprodukt aus der Datenbank zu wählen.

7. Das Hautproduktempfehlungssystem gemäss Anspruch 6, wobei das gewählte Hautpflegeprodukt eine kosmetische Grundlage umfasst.

## Revendications

1. Système de détermination de couleurs cosmétiques (200) comprenant : un collecteur de lumière (202) présentant une sensibilité significative dans les longueurs d'onde rouge et verte visibles et une sensibilité davantage significative dans les longueurs d'onde supérieures à 700 nm, et un composant analytique (290) qui utilise les données de longueurs d'onde visibles issues du collecteur de lumière (202) afin de déterminer une couleur de cheveu cosmétique, **caractérisé en ce que** le composant analytique (290) utilise également des données d'infrarouges issues du collecteur de lumière (202) afin de déterminer une couleur de cheveu cosmétique.

2. Système selon la revendication 1, dans lequel le collecteur de lumière (202) présente la sensibilité significative dans les longueurs d'onde rouge et verte visibles et dans les longueurs d'onde supérieures à 800 nm.

3. Système selon la revendication 1 ou 2, dans lequel le collecteur de lumière (202) présente également une sensibilité significative dans les longueurs d'onde bleues visibles, et le composant analytique (290) utilise les données de longueurs d'onde visibles et d'infrarouges lors de la détermination de la couleur de cheveu.

4. Système selon l'une des revendications précédentes, comprenant en outre un mécanisme destiné à surpondérer des longueurs d'onde particulières ou des plages de longueurs d'onde particulières lors de la détermination de la couleur de cheveu.

5. Système de recommandation de produit pour les cheveux, qui comprend le système de détermination de couleurs cosmétiques selon l'une des revendications 1 à 4, une base de données de produits de coloration pour les cheveux, et un logiciel qui utilise les informations issues du système de détermination de couleurs cosmétiques afin de sélectionner un produit de coloration pour les cheveux à partir de la base de données.

6. Système de recommandation de produit pour la peau, qui comprend le système de détermination de couleurs cosmétiques selon l'une des revendications 1 ou 2, une base de données de produits de soins pour la peau, et un logiciel qui utilise les informations issues du système de détermination de couleurs cosmétiques afin de sélectionner un produit de soins pour la peau à partir de la base de données.

7. Système de recommandation de produit pour la peau selon la revendication 6, dans lequel le produit de soins pour la peau sélectionné comprend une base cosmétique.
